# EUROPEAN PATENT APPLICATION

(11) **EP 4 248 939 A1**
(43) Date of publication of application: **27.09.2023**
(21) Application number: 21859369.7
(22) Date of filing: 15.12.2021
(51) Int. Cl.: A61K 8/22, C11C 3/00, C11B 3/08, B01J 10/00

(54) **OZONISED OIL PRODUCTION METHOD, REACTOR AND OIL PRODUCED**

(30) Priority: 29.01.2021 ES 202130078
(71) Applicant: Keybiological S.L., 36214 Vigo (ES)
(72) Inventor: FERNANDEZ ROGDRIGUEZ, Juan Luis, (ES); GARCIA KIRSTEN, Jorge, (ES)
(74) Representative: Clarke, Modet y Cía., S.L.
(86) International application number: PCT/IB2021/000944
(87) International publication number: WO 2022/162412

(57) **Abstract**

A method and a reactor for the production of ozonised oil from olive oil or other oils or fats at 9-12 °C. The oil is treated by bubbling ozone inside a chamber (1), using a membrane diffuser, and all surplus ozone is destroyed. Ozone concentration is 250-300 g/Nm³, which is achieved by cold plasma generation. The oil thus produced has improved properties, with respect to the health of people, for the treatment of pathologies, and is not degraded.

## Description

### FIELD OF THE INVENTION

The present invention refers to an ozonised oil production method, the reactor and the oil produced. Ozonisation is a process of hyperoxidation of the unsaturated fatty acids present in vegetable or animal oils or fats using ozone to obtain a whole range of peroxidic products (hydroperoxides, ozonides, diperoxides, peroxides and polyperoxides), which are responsible for the broad biological activity of these ozonised oils.

It is applied in the treatment of oils and fats for making pharmaceutical or cosmetic products in the human and animal food industries. Its use in biocides and topical- and oral-use medicines is also envisaged.

### STATE OF THE ART

The treatment of vegetable oils and fats using ozone was born with patent US984722 (Twombly), wherein coconut oil was ozonised at 28 °C. Other methods with their patents were subsequently developed, such as US1210949, with rapeseed oil, US2356062, with olive oil which allowed ozonisation levels to be adjusted, and US3504038, which produced aldehydes by reducing the oil with hydrogen.

European application EP2025740 is also particularly relevant as its invention performs ozonisation at temperatures similar to the invention. This method, however, greatly affects the oil, which even turns into a gel. The resulting product is an oxidant, as mentioned in paragraph [30] thereof.

KR20170057515A discloses a second method for generating a cream by ozonisation, at a temperature lower than the invention and using an ultrasonic diffuser.

WO2003085072A1 is considered to give a description of the state of the art that is particularly suitable for understanding the current situation. The reader is thus referred to this patent to complete the description of the state of the art.

Patent ES251979T3, to Ozonator, concerning a method for the cold production (20 °C) of ozone for water ozonisation, is considered relevant.

The applicant hereof is not aware of any oil ozonisation method or reactor with characteristics similar to the invention that would make it possible to obtain such an advantageous oil.

### BRIEF DESCRIPTION OF THE INVENTION

The invention consists in an ozonised oil production method according to the claims. It also refers to the reactor and to the oil thus produced.

The process of oxidation of vegetable or animal oil or fats with ozone of the invention is carried out in the liquid state of the fatty acid, at a temperature between 9 °C and 12 °C and atmospheric pressure. The integrity of the oil and the achievement of the hyperoxidation process are thus ensured without any degradation of the fatty acid owing to the temperature. The oil may be edible (olive, sunflower...) or inedible depending on the intended end use.

The oil or fat to be treated with the method may have been previously used for cooking, so it will be burnt. Thanks to the method, however, a highly peroxidised fat or oil is obtained having extended characteristics relative to the starting used oil, which increases the recirculation of this waste. The method thus makes it possible to use this waste to obtain an oil with antiseptic, healing and regenerating properties, ideal for manufacturing creams and soaps for dermo-cosmetic use and also as an ingredient in natural biocides, owing to the fact that a very high peroxidation index is achieved.

Fish oil, which is rich in omega-3 fatty acids, can also be used; despite the fact of being less unsaturated, a very high peroxidation index is achieved.

The method is performed with a cold plasma ozone generator, using an anodised aluminium reactor. A preferred model is the one marketed by Primozone under the GM1-4 3.0 trade name. This use lowers the need for an enclosed, air-conditioned space for the generator, which results in a smaller system footprint and lowers the consumption of energy. This temperature control efficiency also improves ozone production, as high temperatures have a negative impact on ozone formation and contribute to the production of ionic contaminants. Moreover, a higher ozone concentration can be achieved, leading to a perfect oxidation of the oil or fat. It allows ozone concentrations above 250 g/Nm³ (grams per normal cubic metre) to be achieved. In the method, concentrations of 250-300 g/Nm³ in the injected gas will be used to achieve the desired level of peroxidation. The ozone generator used is designed and marketed for the ozonisation of large quantities of water. It is therefore necessary to make an adjustment and a parametrisation that are suitable for the new use by setting the automatic gas diffusion control according to the evolution of the density, the viscosity, the pH and other variables of the oil or fat being treated.

Thus, the result of the method is a liquid containing trioleins (9-oxononanoic acid) - also called triolefins, which has not been found in the prior art. They are the main component of human body fat and a major component of human skin fats. These fatty acids are the best for regenerating, moisturising, nourishing, and soothing the skin as they are the same oils that are naturally generated by our skin. An oil with a peroxidation index (IP) ranging from 400 to 1500 mEqO₂/kg is obtained instead of an oil with an index of 10 mEqO₂/kg, as obtained in EP2025740.

The resulting product is oxygenating for the skin by giving up oxygen to the cellular epithelia instead of oxidising the skin, as indicated in previous products (EP2025740). In addition, its disinfecting action ensures that microorganisms of an external origin are eliminated and promotes the regulation of the naturally occurring flora.

This method allows compounds such as azelaic acid or triolefins which are not produced in other temperature, concentration, or pressure ranges to be produced. As an additional advantage, ozonisation occurs in the longer chains, so the resulting molecule is nonvolatile. In other words, no volatile peroxides appear; only large-molecule peroxides appear, with clear functions, which demonstrates a much greater stabilisation of the product with respect to other processes (eg the aforementioned triolefins) despite high peroxidation indices being attained. The absence of volatiles modifies the smell since the product loses the "burnt" aroma of other ozonisated oils.

According to studies published by Saravanan et al, azelaic acid appears to be essential in increasing liver enzyme activity to restore glucose levels (experiment on diabetic mice). Its use as a cosmetic is also known. 9-oxononanoic acid decreases lipogenesis in rat liver (Natake et al).

The method produces a toxic-free product when the starting component is edible, and the desired antiseptic compounds.

The ozonised oil production method is carried out at 9-12 °C, so the compounds in the oil or fat - typically olive oil - do not degrade. Hence, the phenolic content and antioxidants are not lost. In addition, to prevent the production of other undesirable compounds, when the system has surplus ozone, it is not recirculated but destroyed after passing through the oil. It is likewise possible to control the flow and concentration of ozone so that there is no ozone surplus (measured by sensors at the reactor's outlet). The method is carried out in batches to ensure that the result is completely homogeneous and has a very fine control of the temperature, thus ensuring that the correct temperature is maintained at all times. For example, by cooling the ozone input stream, which in any case enters the reactor at a very low temperature.

The oil ozonisation reactor that carries out this method is made up of a chamber with a bubbler (eg a membrane bubbler), which introduces ozone into the oil or fat from an external generator. In addition, it has means for regulating the temperature and a destructor of surplus ozone. The process is exhaustively controlled by means of software, which controls the temperature, pressure, surplus ozone, diffusion rate and bubbling and constantly and instantaneously adjusts the ozonisation process during production as the treated oil or fat evolves.

For example, a security check is also carried out: The process has automated leakage, temperature, diffusion process and tank pressure control to ensure an automated shutdown of the process. At this point, light and sound warnings would go off.

The membrane diffuser is comprised of ultra-fine bubble diffusion modules which have a high chemical or fouling resistance and are made of eg environmentally friendly polypropylene. An example of a usable diffuser is the AFD 270 model marketed by SSI Aeration Inc., New York (USA), which has 176,000 holes per square metre. Each diffusion module has been verified to have a perfect hole depth to ensure a uniform release of air, which provides a remarkable efficiency in the transfer of ozone, with a minimal pressure drop. The diffusion modules have low shrinkage and hardening, but sufficient to prevent slippage and withstand temperatures up to 100 °C. In addition, the aeration of the clean pipe system is maintained by means of several built-in check valves. Like the ozone generator, this diffuser is designed for treatment of water, not oils or fats.

Other variants will be discussed below.

### DESCRIPTION OF THE DRAWINGS

A series of figures are provided to facilitate the understanding of the invention:

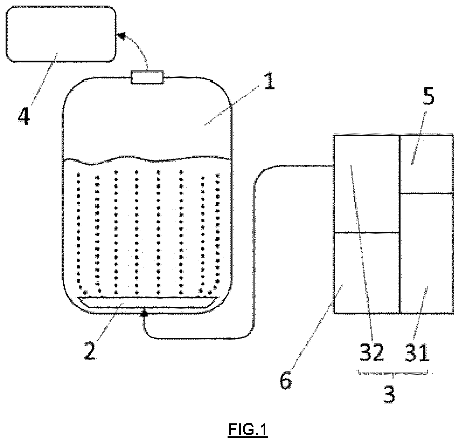

Figure 1 is a diagram of an example of a reactor for the detailed description of the invention.

### DETAILED DESCRIPTION OF THE INVENTION

An embodiment of the invention will now be briefly described as an illustrative, non-limiting example thereof.

In the fatty acid ozonisation process according to the invention, oil is treated under the most suitable conditions to obtain a non-degraded product.

The process is carried out starting from olive oil or other vegetable oil in a reactor made up of a chamber (1) with a bubbler (2), preferably of the membrane type, which introduces ozone into the oil. The ozone comes from an external generator (3) and is captured at the outlet of the chamber (1) to be sent to a destructor (4) provided with a UV light, a catalyst, or a burner. Ozone is not recirculated because of the potential synthesis of substances that are harmful for human and/or animal health. This generator (3) is a cold plasma generator and achieves a concentration of 250-300 g/Nm³, preferably above 260 g/Nm³.

In the preferred example, the generator (3) is an automatised ozone production plant consisting of one or more - eg two - ambient oxygen concentrators (32) and two refrigerated, industrial ozone generating cores (31). The number of refrigerated industrial ozone generating cores (31) will depend on the desired output. The generator (3) is of the "cold plasma" type, such as the one mentioned in the state of the art, which achieves a gas concentration of 250-300 g/Nm³.

The method is carried out in the liquid state of the oil, at a temperature of 9-12 °C, at atmospheric pressure.

The temperature of the process, during the generation of ozone, and inside the chamber is controlled. It is also necessary to control the input (mg/h of ozone) and the amount of ozone reaching the ceiling of the chamber (1), which allows the amount of ozone captured by the oil to be known. It is also convenient to control the pressure inside the chamber (1) or tank and the flow of ozonised air. These controls allow the reproducibility and safety of the process to be ensured. The reactor also comprises automated leakage control to ensure the automated shutdown of the process. For example, by means of one or more ozone sensors on the outside of the reactor. All these variables are monitored by a control unit (5) which controls a temperature regulator (6).

Should an anomalous circumstance occur in these parameters, light and sound warnings would be flashed.

The suitable quantity, flow and concentration of ozone at the time of the peroxidation of the oil which is object to the ozonisation process are also closely controlled. Dilution is electronically controlled by means of an automaton piece of equipment that is specifically adjusted to the process. Control is carried out autonomously by the piece of equipment's control unit. The peroxidation that is sought depends on the target properties of the manufactured oils. Peroxide indices range from 100 to 3500.

The process is preferably carried out in batches to ensure homogeneity and stops when the desired peroxide index has been reached or a preset percentage of the introduced ozone is detected as a surplus. The time therefore depends on the oil introduced or the peroxidation target.

The result is in liquid state, with a characteristic smell, a specific viscosity, certain impurities, a specific oleic acidity and a specific peroxidation index according to the purpose of the ingredient. It is not necessary to dilute it in order for it to be used and is readily absorbable at the epithelial level.

The oil with ozone according to the invention remains stable over time; the oil loses 15% of its peroxidation index per year at room temperature, the loss decreasing at lower temperatures.

First example: starting from an extra virgin olive oil, and after carrying out ozonisation (conditions: ozone concentration 275 g/Nm³, with a flow rate of 79 g/h and a dwell time of 66 h, up to an ozonisation rate of 508 mEq/kg), the presence of azelaic acid and 9-oxonononanoic acid could be detected [by mass spectrometry; 2000 ppm of product to be analysed with isopropanol/acetonitrile solvent (80:20 ratio) and 0,1 % of formic acid and a flow rate of 40 µl/min].

Second example: 7.5 litres of used cooking oil treated for 43 h, with an ozone concentration of 250 g/Nm³ and flow rate of 40 g/h. A peroxidation index of 823.92 mEq/kg was attained.

Third example: fish oil (10 I), treated for 187 h, with an ozone concentration of 250 g/Nm³ and a flow rate 40 g/h. A PI of 722.83 mEq/kg is reached.

All materials are ozone-resistant.

## Claims

1. A method for the production of ozonised oil from vegetable or animal oil or fat, **characterised in that** it is carried out at 9-12 °C and at atmospheric pressure and with an ozone flow with a concentration between 250 and 300 g/Nm³.

2. A method for the production of ozonised oil according to claim 1, **characterised in that** the ozone is generated by means of cold plasma.

3. A method for the production of ozonised oil according to claim 1, **characterised in that** the method is carried out in batches.

4. A method for the production of ozonised oil according to claim 1, **characterised in that** it comprises the destruction of all surplus ozone.

5. A method for the production of ozonised oil according to claim 1, **characterised in that** it starts from used cooking oil.

6. A method for the production of ozonised oil according to claim 1, **characterised in that** it starts from fish oil.

7. An ozonised oil, **characterised in that** it has been produced with the method of any one of the preceding claims.

8. An ozonised oil according to claim 7, **characterised in that** it comprises azelaic acid.

9. An ozonised oil according to claim 7, **characterised in that** it comprises triolefins.

10. An oil ozonisation reactor for the application of the method of claim 1, which is made up of a chamber (1) with a bubbler (2), which introduces ozone into the oil from an external generator (3), **characterised in that** it comprises a destructor (4) of surplus ozone.

11. A reactor according to claim 10, **characterised in that** the bubbler (2) is of the membrane type.

12. A reactor according to claim 10, **characterised in that** it comprises an automated ozone leakage control.

13. A reactor according to claim 10, **characterised in that** the generator (3) is an automaton ozone production plant consisting of one or more ambient oxygen concentrators (32) and one or more refrigerated cores (31) for the generation of industrial ozone by means of cold plasma.

14. A reactor according to claim 10, **characterised in that** it comprises controlling the temperature, pressure, surplus ozone, diffusion rate and bubbling.
